(19) **Europäisches Patentamt**
**European Patent Office**
**Office européen des brevets**

(11) **EP 3 127 548 B1**

(12) **EUROPEAN PATENT SPECIFICATION**

(45) Date of publication and mention
of the grant of the patent:
**08.05.2019 Bulletin 2019/19**

(21) Application number: **15773245.4**

(22) Date of filing: **23.03.2015**

(51) Int Cl.:
*A61K 8/9789* (2017.01)     *A61Q 19/02* (2006.01)
*A61K 8/02* (2006.01)     *A61Q 19/00* (2006.01)

(86) International application number:
**PCT/KR2015/002841**

(87) International publication number:
**WO 2015/152552 (08.10.2015 Gazette 2015/40)**

(54) **COMPOSITION COMPRISING EXTRACT OF ARTEMISIA UMBELLIFORMIS**

ZUSAMMENSETZUNG MIT EINEM EXTRAKT AUS ARTEMISIA UMBELLIFORMIS

COMPOSITION COMPRENANT UN EXTRAIT D'ARTEMISIA UMBELLIFORMIS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.03.2014 KR 20140037504**

(43) Date of publication of application:
**08.02.2017 Bulletin 2017/06**

(73) Proprietor: **Amorepacific Corporation
Seoul 140-777 (KR)**

(72) Inventors:
• **PARK, Pil Joon
Yongin-si
Gyeonggi-do 446-729 (KR)**
• **LEE, So Hee
Yongin-si
Gyeonggi-do 446-729 (KR)**
• **CHO, Eun Gyung
Yongin-si
Gyeonggi-do 446-729 (KR)**
• **LEE, Tae Ryong
Yongin-si
Gyeonggi-do 446-729 (KR)**

(74) Representative: **Novagraaf Technologies
Bâtiment O2
2, rue Sarah Bernhardt
CS90017
92665 Asnières-sur-Seine Cedex (FR)**

(56) References cited:
**WO-A1-2009/088109     WO-A2-2012/007670
CH-A5- 696 135     KR-A- 20120 113 459
KR-A- 20130 082 063**

• **PATRIZIA RUBIOLO ET AL: "Chemical and
Biomolecular Characterization of Artemisia
umbelliformis Lam., an Important Ingredient of
the Alpine Liqueur "Genepì"", JOURNAL OF
AGRICULTURAL AND FOOD CHEMISTRY, vol.
57, no. 9, 13 May 2009 (2009-05-13), pages
3436-3443, XP055229287, ISSN: 0021-8561, DOI:
10.1021/jf803915v**
• **'DSM ALPAFLOR RANGE.' DSM NUTRITIONAL
PRODUCTS 2013, XP055359178 Retrieved from
the Internet:
<URL:http://www.sellcare.ch/index_en.php?TP
L=10070>**
• **YAMADA, M. ET AL.: 'Melanin biosynthesis
inhibitors from Tarragon Artemisia dracunculus.'
BIOSCIENCE BIOTECHNOLOGY AND
BIOCHEMISTRY vol. 75, no. 8, 2011, pages 1628
- 1630, XP018503660**
• **VALLES, J. ET AL.: 'The genus Artemisia and its
allies: phylogeny of the subtribe Artemisiinae
(Asteraceae, Anthemideae) based on nucleotide
sequences of nuclear ribosomal DNA internal
transcribed spacers (ITS).' PLANT BIOLOGY. vol.
5, no. 3, 2003, pages 274 - 284, XP055229276**

**Description**

[Technical Field]

The present disclosure relates to a composition comprising an alpine wormwood extract.

[Background Art]

Melanin is a pigment that determines skin color in humans. The skin color is determined by the amount, type and distribution of the produced melanin. The melanin are synthesized and secreated from melanocytes located in the bottom layer of the epidermis and are lost after moving toward the keratinous layer as a result of skin metabolism. The number of melanocytes is almost constant regardless of skin color but the difference in the amount, type and distribution of the produced melanin results in different skin colors.

In the skin, tyrosine is converted to DOPA by the enzyme called tyrosinase, which is finally turned into the blackish brown polymer melanin through a series of oxidation processes.

Melanin is produced by melanocytes located in the basal layer of the skin. It is known that the melanin production is promoted by stimuli such as UV, inflammation, etc. Therefore, by reducing external stimulation, interrupting signal transduction, suppressing the synthesis of the melanin-producing enzyme tyrosinase or inhibiting its activity, the production of melanin can be decreased.

At present, it is known that kojic acid, hydroquinone, arbutin, azelaic acid, aloesin, 4-butylresorcinol, resveratrol, ceramide, sphingosine-1-phosphate, sphingosylphosphorylcholine, etc. can control the melanin production by promoting the degradation of tyrosinase or by regulating its glycosylation. However, their utility is not so high because of unsatisfactory skin whitening effect, low stability and skin irritation. Accordingly, there is a need of a substance which exhibits superior skin whitening effect with few side effects.

Patent document CH 696135 discloses a skin protective composition comprising an extract of Artemisia umbelliformis. Patent document WO 2009/088109 discloses a skin whitening composition comprising diosgenin as an active ingredient.

**[Disclosure]**

[Technical Problem]

The present disclosure is directed to providing a composition having a superior skin whitening effect.

[Technical Solution]

In an aspect, the present disclosure provides a composition for skin whitening, which comprises an extract of alpine wormwood (*Artemisia umbelliformis*) belonging to the genus *Artemisia* as an active ingredient.

[Advantageous Effects]

In an aspect, the composition for skin whitening of the present disclosure, which comprises an alpine wormwood (*Artemisia umbelliformis*) extract as an active ingredient, exhibits skin whitening effect by reducing melanin production and inhibiting tyrosinase activity while having stability without negatively affecting cells. Accordingly, it can be widely used for a composition for extremal application to skin, a cosmetic composition and a pharmaceutical composition.

[Brief Description of Drawings]

FIG. 1 shows a graph showing the result of testing the decrease in melanin content by an alpine wormwood (*Artemisia umbelliformis*) extract according to an aspect of the present disclosure as well as a photograph of test tube taken before the measurement.

FIG. 2 shows a result of testing the decrease in tyrosinase activity by an alpine wormwood (*Artemisia umbelliformis*) extract according to an aspect of the present disclosure.

[Best Mode]

Korean Patent Application No. 10-2014-0037504 filed on March 31, 2014 is incorporated in the present disclosure by reference in its entirety. Also, this application claims all benefits accruing from Korean Patent Application No. 10-2014-0037504 which is incorporated in the present disclosure by reference in its entirety.

Hereinafter, specific exemplary embodiments of the present disclosure will be described in detail, so that those of ordinary skill in the art to which the present disclosure belongs can easily carry out the present disclosure.

In an aspect, the present disclosure may relate to a composition comprising an alpine wormwood (*Artemisia umbelliformis*) extract as an active ingredient.

In an aspect of the present disclosure, the composition may be a composition for skin whitening.

In an aspect of the present disclosure, the composition according to the present disclosure may be a pharmaceutical, food or cosmetic composition. And specifically, in an aspect of the present disclosure, the composition according to the present disclosure may be a composition for extremal application to skin.

In an aspect, the present disclosure may relate to a method for skin whitening, which comprises administering an alpine wormwood (*Artemisia umbelliformis*) extract to a subject in need of skin whitening. Specifically, the administering may be achieved by an administration method described in the present disclosure.

In an aspect, the present disclosure may relate to a use of an alpine wormwood (*Artemisia umbelliformis*) extract for skin whitening.

In an aspect, the present disclosure may relate to an alpine wormwood (*Artemisia umbelliformis*) extract for use in skin whitening.

In an aspect, the present disclosure may relate to a composition for reducing melanin, which comprises an alpine wormwood (*Artemisia umbelliformis*) extract as an active ingredient.

In an aspect, the present disclosure may relate to a method for reducing melanin, which comprises administering an alpine wormwood (*Artemisia umbelliformis*) extract to a subject in need of melanin reduction. Specifically, the administering may be achieved by an administration method described in the present disclosure.

In an aspect, the present disclosure may relate to a use of an alpine wormwood (*Artemisia umbelliformis*) extract for melanin reduction.

In an aspect, the present disclosure may relate to an alpine wormwood (*Artemisia umbelliformis*) extract for use in melanin reduction.

In an aspect, the present disclosure may relate to a composition for suppressing melanin production, which comprises an alpine wormwood (*Artemisia umbelliformis*) extract as an active ingredient.

In an aspect, the present disclosure may relate to a method for suppressing melanin production, which comprises administering an alpine wormwood (*Artemisia umbelliformis*) extract to a subject in need of suppression of melanin production. Specifically, the administering may be achieved by an administration method described in the present disclosure.

In an aspect, the present disclosure may relate to a use of an alpine wormwood (*Artemisia umbelliformis*) extract for suppressing melanin production.

In an aspect, the present disclosure may relate to an alpine wormwood (*Artemisia umbelliformis*) extract for use in suppression of melanin production.

In an aspect, the present disclosure may relate to a composition for inhibiting tyrosinase activity, which comprises an alpine wormwood (*Artemisia umbelliformis*) extract as an active ingredient.

In an aspect, the present disclosure may relate to a method for inhibiting tyrosinase activity, which comprises administering an alpine wormwood (*Artemisia umbelliformis*) extract to a subject in need of inhibition of tyrosinase activity.

In an aspect, the present disclosure may relate to a use of an alpine wormwood (*Artemisia umbelliformis*) extract for inhibiting tyrosinase activity.

In an aspect, the present disclosure may relate to an alpine wormwood (*Artemisia umbelliformis*) extract for use in inhibition of tyrosinase activity.

In an aspect, the present disclosure may relate to a composition for suppressing pigmentation, which comprises an alpine wormwood (*Artemisia umbelliformis*) extract as an active ingredient.

In an aspect, the present disclosure may relate to a composition for treating, improving or preventing pigmentation disorder, which comprises an alpine wormwood (*Artemisia umbelliformis*) extract as an active ingredient. Specifically, in an aspect, the present disclosure may relate to a pharmaceutical composition for treating or preventing pigmentation disorder or a food composition for improving or preventing pigmentation disorder, which comprises an alpine wormwood (*Artemisia umbelliformis*) extract as an active ingredient.

In an aspect, the present disclosure may relate to a method for treating, improving or preventing pigmentation disorder, which comprises administering an alpine wormwood (*Artemisia umbelliformis*) extract to a subject in need of treatment, improvement or prevention of pigmentation disorder. Specifically, the administering may be achieved by an administration method described in the present disclosure.

In an aspect, the present disclosure may relate to a use of an alpine wormwood (*Artemisia umbelliformis*) extract for treating, improving or preventing pigmentation disorder.

In an aspect, the present disclosure may relate to an alpine wormwood (*Artemisia umbelliformis*) extract for use in treatment, improvement or prevention of pigmentation disorder.

In an aspect of the present disclosure, the term pigmentation disorder is used in a broad concept, including all the pigmentation disorders caused by various causes. Specifically, it may refer to pigmentation disorder occurring in skin, but is not limited thereto. Specifically, in an aspect of the present disclosure, the pigmentation disorder may include senile spots, inflammatory pigmentation disorder, freckles, acquired dermal melanosis, hyperpigmentation, etc., but is not limited thereto.

In the present disclosure, the term "pigmentation" or "hyperpigmentation" refers to a skin damage characterized by actual or perceived, excessively dark skin color. The skin damage may be actual and dark skin areas may appear due to such conditions as aging, excessive exposure to sunlight or diseases. The dark skin area may be in the form of spots, blotches or relatively large dark areas. The skin damage may also be one perceived by individuals who have cosmetic desire to have light skin.

Accordingly, the composition of the present disclosure is useful for various types of skin pigmentation such as melanoma, for example, in removing pigmented dark patches on the face or other parts of the body or inducing spontaneous whitening of pigmented skin. Typically, the dark skin damage is caused by increased melanin level. The composition according to the present disclosure may be used to treat hyperpigmentation, i.e., to lighten the dark skin or prevent hyperpigmentation, or to prevent skin darkening by reducing or preventing excessive melanin production.

In an aspect of the present disclosure, the concentration of the alpine wormwood (*Artemisia umbelliformis*) extract in the composition comprising the same may be 0.1-10 ppm based on the total volume of the composition, although not being limited thereto. Specifically, in an aspect of the present disclosure, the concentration of the alpine wormwood (*Artemisia umbelliformis*) extract in the composition comprising the same may be 0.01 ppm or higher, 0.1 ppm or higher, 0.5 ppm or higher, 0.6 ppm or higher, 0.7 ppm or higher, 0.8 ppm or higher, 0.9 ppm or higher, 1.0 ppm or higher, 1.1 ppm or higher, 1.2 ppm or higher, 1.3 ppm or higher, 1.4 ppm or higher, 1.5 ppm or higher, 1.6 ppm or higher, 1.7 ppm or higher, 1.8 ppm or higher, 1.9 ppm or higher, 2.0 ppm or higher, 2.1 ppm or higher, 2.2 ppm or higher, 2.3 ppm or higher, 2.4 ppm or higher, 2.5 ppm or higher, 2.6 ppm or higher, 2.7 ppm or higher, 2.8 ppm or higher, 2.9 ppm or higher, 3 ppm or higher, 3.2 ppm or higher, 3.4 ppm or higher, 3.6 ppm or higher, 3.8 ppm or higher, 4.0 ppm or higher, 4.5 ppm or higher, 5.0 ppm or higher, 5.5 ppm or higher, 6.0 ppm or higher, 7.0 ppm or higher, 8.0 ppm or higher, 9.0 ppm or higher, 10.0 ppm or higher, 50 ppm or higher, 100 ppm or higher, 500 ppm or higher or 1000 ppm or higher, although not being limited thereto, and may be 2000 ppm or lower, 1000 ppm or lower, 500 ppm or lower, 100 ppm or lower, 50 ppm or lower, 15.0 ppm or lower, 10.0 ppm or lower, 8.0 ppm or lower, 6.0 ppm or lower, 5.5 ppm or lower, 5.0 ppm or lower, 4.5 ppm or lower, 4.0 ppm or lower, 3.8 ppm or lower, 3.6 ppm or lower, 3.4 ppm or lower, 3.2 ppm or lower, 3.0 ppm or lower, 2.8 ppm or lower, 2.6 ppm or lower, 2.4 ppm or lower, 2.2 ppm or lower, 2.0 ppm or lower, 1.8 ppm or lower, 1.6 ppm or lower, 1.4 ppm or lower, 1.2 ppm or lower, 1.0 ppm or lower, 0.8pm or lower, 0.6 ppm or lower, 0.4 ppm or lower or 0.2 ppm or lower, based on the total volume of the composition.

In an aspect of the present disclosure, the alpine wormwood (*Artemisia umbelliformis*) may be one or more selected from a group consisting of the leaf, flower, stem and root of the plant alpine wormwood (*Artemisia umbelliformis*).

In an aspect of the present disclosure, the alpine wormwood (*Artemisia umbelliformis*) extract may be prepared by a method comprising (1) a step of extracting alpine wormwood (*Artemisia umbelliformis*) with water, an organic solvent or a combination thereof.

In an aspect of the present disclosure, the method may further comprise, prior to the step (1), a step of processing alpine wormwood (*Artemisia umbelliformis*). Specifically, the processing may be drying and pulverizing alpine wormwood (*Artemisia umbelliformis*). However, without being limited thereto, any processing for facilitating the extraction is comprised. Specifically, the drying may be sunlight drying, hot air drying, evaporation drying, spray drying or freeze drying, more specifically hot air drying. On the other hand, in an aspect of the present disclosure, live alpine wormwood (*Artemisia umbelliformis*) may be extracted as it is without any processing.

In an aspect of the present disclosure, the method may further comprise a step of removing the solvent by distillation following the extraction. Specifically, the distillation may be vacuum distillation.

In an aspect of the present disclosure, the method may further comprise a step of adding one or more of glycerin and a preservative to the resulting concentrate following the distillation.

In an aspect of the present disclosure, the method may further comprise a step of filtering following the solvent removal or following the addition of one or more of glycerin and a preservative.

In an aspect of the present disclosure, the alpine wormwood (*Artemisia umbelliformis*) extract may be an extract of one or more selected from a group consisting of water, an organic solvent and a mixture thereof. Specifically, in an aspect of the present disclosure, the organic solvent may be one or more selected from a group consisting of a $C_1$-$C_6$ lower alcohol, butylene glycol and propylene glycol. More specifically, the lower alcohol may be ethanol.

Melanocytes used in the present disclosure may be the melanocytes disclosed in PCT/KR2012/007815 or Korean Patent Application No. 10-2011-0099728 (keratinocyte-adapted melanocytes, KaMC). The melanocytes used in the present disclosure may be obtained according to the method and procedure disclosed in the references and may also be cultured according to the method disclosed in the references. The melanocytes were deposited in the Korean Collection for Type Culture (KCTC) of Korea Research Institute of Bioscience and Biotechnology on Sep. 14, 2011 under the Budapest Treaty and were given the accession number KCTC 12015BP.

In the present disclosure, "skin" refers to the organ covering the surface of an organism. It is composed of the epidermis, the dermis and the subcutaneous fat layer and is used in the broadest concept, including not only the tissues covering the face or the entire outer part of the body but also the scalp and hair.

In the present disclosure, "alpine wormwood (*Artemisia umbelliformis*)" also known as White Genepi or Genepi Blanco refers to a herb belonging to the genus *Artemisia* of the family *Asteraceae.* Alpine wormwood is used for the production of the liquor Genepi and the leaves are used in the preparation of tea or condiment.

In the present disclosure, "extract" refers to a substance extracted from a natural product, regardless of the extraction method, extraction solvent, extracted components or the type of the extract. It is used in a broad concept, including any substance that can be obtained by processing or treatment of a substance extracted from a natural product. Specifically, the processing or treatment may be fermenting or enzymatically treating the extract. Accordingly, in the present disclosure, the extract is used in a broad concept, including a fermentation product, a concentrate and a dried product. Specifically, in the present disclosure, the extract may be a fermentation product.

In the present disclosure, "alpine wormwood (*Artemisia umbelliformis*) extract" includes any substance obtained by extracting alpine wormwood (*Artemisia umbelliformis*), regardless of the extraction method, extraction solvent, extracted components or the type of the extract. It includes a substance obtained by an extraction method including a process treating with heat, an acid, a base, an enzyme, etc. and is used in a broad concept, including any substance that can be obtained by processing or treatment of a substance extracted from alpine wormwood (*Artemisia umbelliformis*). Specifically, the processing or treatment may be additional fermenting or additional enzymatic treating the alpine wormwood (*Artemisia umbelliformis*) extract. Accordingly, the alpine wormwood (*Artemisia umbelliformis*) extract of the present disclosure may be a fermentation product.

In an aspect of the present disclosure, "alpine wormwood (*Artemisia umbelliformis*)" may be an extract, live alpine wormwood (*Artemisia umbelliformis*), a pulverization product of live alpine wormwood (*Artemisia umbelliformis*), a dried product of live alpine wormwood (*Artemisia umbelliformis*), a dried pulverization product of live alpine wormwood (*Artemisia umbelliformis*) or a fermentation product alpine wormwood (*Artemisia umbelliformis*), although not being limited thereto. And, the alpine wormwood (*Artemisia umbelliformis*) used in the present disclosure is not limited in the way how it is obtained. It may be either cultivated or purchased commercially. Also, all or part of its areal part or root part may be used. More specifically, one or more selected from a group consisting of the leaf, stem, root and flower of the plant alpine wormwood (*Artemisia umbelliformis*) may be used. The alpine wormwood (*Artemisia umbelliformis*) of the present disclosure is not necessarily dried and may be in any form as long as the active ingredients of alpine wormwood (*Artemisia umbelliformis*) can be adequately extracted.

In an aspect of the present disclosure, the water includes distilled water or purified water, and the organic solvent includes one or more selected from a group consisting of an alcohol such as a $C_1$-$C_6$ lower alcohol, acetone, ether, ethyl acetate, diethyl ether, methyl ethyl ketone and chloroform, although not being limited thereto.

In an aspect of the present disclosure, the alpine wormwood (*Artemisia umbelliformis*) extract may include a $C_1$-$C_6$ alcohol extract of alpine wormwood (*Artemisia umbelliformis*). Specifically, the alcohol may be methanol or ethanol.

In an aspect of the present disclosure, the alpine wormwood (*Artemisia umbelliformis*) extract may be obtained by a method including a step of extracting alpine wormwood (*Artemisia umbelliformis*) with water, an organic solvent or a mixture thereof.

In an aspect of the present disclosure, the alpine wormwood (*Artemisia umbelliformis*) extract may be a crude extract of a solvent selected from a group consisting of water, an organic solvent and a combination thereof. The organic solvent may be a $C_1$-$C_6$ alcohol. Specifically, the $C_1$-$C_6$ alcohol may be methanol or ethanol. In an aspect of the present disclosure, when the alpine wormwood (*Artemisia umbelliformis*) is extracted with a solvent, the solvent may be added in an amount of about 5-15 times that of the alpine wormwood (*Artemisia umbelliformis*). Specifically, solvent may be added in an amount of about 10 times that of the alpine wormwood (*Artemisia umbelliformis*), although not being limited thereto.

In an aspect of the present disclosure, the extraction may be performed by hot water extraction, ethanol extraction, heating extraction, cold extraction, reflux extraction, reflux condensation extraction, ultrasonic extraction, etc. Any extraction method known to those skilled in the art may be used without limitation. Specifically, the extraction may be performed by hot water extraction or ethanol extraction.

In an aspect of the present disclosure, the extraction may be performed at room temperature. However, for more effective extraction, it may be performed at elevated temperatures. The extraction may be performed preferably at about 40-100 °C, more preferably at about 80 °C, although not being limited thereto. The extraction may be performed for about 2-14 hours, specifically for about 8-14 hours, more specifically for about 11-13 hours, most specifically for 12 hours. However, the extraction time is not limited thereto and may vary depending on such conditions as extraction solvent, extraction temperature, etc. The extraction may be performed more than once in order to obtain a larger amount of the active ingredients. The extraction may be performed continuously preferably 1-5 times, more preferably 3 times, and the resulting extracts may be combined.

In an aspect of the present disclosure, the alpine wormwood (*Artemisia umbelliformis*) extract may include the crude extract of alpine wormwood (*Artemisia umbelliformis*) as described above and may also include a soluble fraction of an organic solvent with low polarity, which is obtained by further extracting the crude extract with the organic solvent. In an aspect of the present disclosure, the organic solvent may be hexane, methylene chloride, ethyl acetate, n-butanol, etc., although not being limited thereto. The extract or the soluble fraction of the extract may be used as it is, after being filtered and then concentrated, or after being concentrated and dried.

In an aspect of the present disclosure, the drying may be evaporation drying, spray drying or freeze drying. Specifically, the freeze drying may be performed at - 50 to -70 °C for 3-4 days.

Formulations of the cosmetic composition according to an aspect the present disclosure are not particularly limited but may be selected properly depending on purposes. For example, the composition may be prepared into one or more formulation selected from a group consisting of a skin lotion, a skin softener, a skin toner, an astringent lotion, a milk lotion, a moisturizing lotion, a nourishing lotion, a massage cream, a nourishing cream, a moisturizing cream, a hand cream, a foundation, an essence, a nourishing essence, a pack, a soap, a cleansing foam, a cleansing lotion, a cleansing cream, a body lotion and a body cleanser, although not being limited thereto.

When the cosmetic composition according to the present disclosure is formulated as a paste, a cream or a gel, an animal fiber, a plant fiber, wax, paraffin, starch, tragacanth, a cellulose derivative, polyethylene glycol, silicone, bentonite, silica, talc, zinc oxide, etc. may be used as a carrier component.

When the cosmetic composition according to the present disclosure is formulated as a powder or a spray, lactose, talc, silica, aluminum hydroxide, calcium silicate or polyamide powder may be used as a carrier component. In particular, when it is formulated as a spray, it may further comprise a propellant such as chlorofluorohydrocarbon, propane/butane or dimethyl ether.

When the cosmetic composition according to the present disclosure is formulated as a solution or an emulsion, a solvent, a solubilizer or an emulsifier may be used as a carrier component. For example, water, ethanol, isopropanol, ethyl carbonate, ethyl acetate, benzyl alcohol, benzyl benzoate, propylene glycol, 1,3-butylglycol oil, glycerol fatty ester, polyethylene glycol or fatty acid ester of sorbitan may be used.

When the cosmetic composition according to the present disclosure is formulated as a suspension, a diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, etc. may be used as a carrier component.

When the cosmetic composition according to the present disclosure is formulated as a surfactant-containing cleanser, fatty alcohol sulfate, fatty alcohol ether sulfate, sulfosuccinic acid monoester, isethionate, an imidazolinium derivative, methyl taurate, sarcosinate, a fatty acid amide ether sulfate, an alkyl amidobetaine, a fatty alcohol, a fatty acid glyceride, a fatty acid diethanolamide, a vegetable oil, a lanolin derivative, an ethoxylated glycerol fatty acid ester, etc. may be used as a carrier component.

The cosmetic composition according to the present disclosure may further comprise a functional additive and ingredients commonly used in a cosmetic composition, in addition to the alpine wormwood (*Artemisia umbelliformis*) extract. The functional additive may include those selected from a group consisting of a water-soluble vitamin, an oil-soluble vitamin, a polypeptide, a polysaccharide, a sphingolipid and a seaweed extract.

If necessary, the cosmetic composition of the present disclosure may further comprise, in addition to the functional additive, the ingredients commonly used in a cosmetic composition. Examples of the further comprised ingredients may include an oil, a fat, a humectant, an emollient, a surfactant, an organic or inorganic pigment, an organic powder, a UV absorbent, a preservative, a sterilizer, an antioxidant, a plant extract, a pH control agent, an alcohol, a colorant, a flavor, a blood circulation promoter, a cooling agent, an antiperspirant, purified water, etc.

In an aspect, the present disclosure also relates to a composition for external application to skin, which comprises an alpine wormwood (*Artemisia umbelliformis*) extract as an active ingredient. The composition for external application to skin refers to any type of composition that can be applied from outside the skin and various types of cosmetics may be included therein.

The pharmaceutical composition according to the present disclosure may be in the form of various oral or parenteral formulations. When the composition is formulated, a commonly used diluent or excipient such as a filler, an extender, a binder, a wetting agent, a disintegrant, a surfactant, etc. is used. Solid formulations for oral administration include a tablet, a pill, a powder, a granule, a soft or hard capsule, etc., and these solid formulations may be prepared by mixing with at least one excipient such as starch, calcium carbonate, sucrose, lactose, gelatin, etc. In addition to the simple excipient, a lubricant such as magnesium stearate, talc, etc. may also be used. Examples of liquid formulations for oral administration include a suspension, a liquid for internal use, an emulsion, a syrup, etc. In addition to a commonly used simple diluent such as water and liquid paraffin, various excipients such as a humectant, a sweetener, an aromatic, a preservative, etc. may also be contained. Formulations for parenteral administration include a sterilized aqueous solution, a non-aqueous solution, a suspension, an emulsion, a lyophilized product and a suppository. The non-aqueous solution or suspension may contain propylene glycol, polyethylene glycol, a vegetable oil such as olive oil, an injectable ester such as ethyl oleate, etc. As a base of the suppository, witepsol, macrogol, tween 61, cocoa butter, laurin butter, glycerogelatin, etc. may be used.

The composition of the present disclosure may comprise a pharmaceutically acceptable salt of the active ingredient, and may be used alone or in combination with another pharmaceutically active compound(s). The salt is not particularly limited as long as it is pharmaceutically acceptable. For example, a salt of hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methanesulfonic acid, benzenesulfonic acid, toluenesulfonic acid, naphthalenesulfonic acid, etc. may be used.

The composition of the present disclosure may be administered parenterally or orally depending on the desired purposes. A daily dose of 0.1-500 mg, specifically 1-100 mg, per kg body weight may be administered once or several times a day. The administration dose for a particular patient may be changed depending on the body weight, age, sex and health conditions of the patient, diet, administration time, administration method, rate of excretion, severity of a disease, and so forth.

The pharmaceutical composition according to the present disclosure may be formulated into any pharmaceutically appropriate form including oral formulations such as a powder, a granule, a tablet, a soft or hard capsule, a suspension, an emulsion, a syrup, an aerosol, etc., formulations for external application to skin such as an ointment, a cream, etc., a suppository, an injection, a sterile solution for injection, etc. according to commonly employed methods. Specifically, it may be formulated into an injection or a formulation for external application to skin.

The composition according to the present disclosure may be administered to mammals including rat, mouse, cattle, human, etc. via various routes including parenteral and oral routes. All types of administration may be expected. For example, it may be administered orally, transdermally, rectally, intravenously, intramuscularly, subcutaneously, intrauterinally or intracerebroventricularly.

The composition according to the present disclosure may be administered via various routes that can be easily selected by those skilled in the art. In particular, the pharmaceutical composition according to the present disclosure may be applied on skin as a formulation for external application to skin.

In an aspect of the present disclosure, the food composition may be a health functional food composition.

Formulations of the food composition according to the present disclosure are not particularly limited. For example, it may be formulated into a tablet, a granule, a powder, a liquid such as a drink, a caramel, a gel, a bar, etc. The food composition of each formulation may comprise, in addition to the active ingredient, ingredients commonly used in the art that can be selected by those skilled in the art without difficulty. In this case, a synergic effect may be achieved.

In the food composition according to the present disclosure, determination of the administration dose of the active ingredient is within the level of those skilled in the art. For example, a daily dosage may be 0.1-5000 mg/kg/day, more specifically, 50-500 mg/kg/day. However, without being limited thereto, the administration dose may vary depending on various factors such as the age and health conditions of the subject, presence of complication(s), etc.

For example, the food composition according to the present disclosure may be various foods such as a chewing gum, a caramel, a candy, a popsicle, confectionery, etc., drinks such as a soft drink, a mineral water, an alcohol beverage, etc. or health functional foods including vitamin, mineral, etc.

In addition, the food composition according to the present disclosure may comprise various nutrients, vitamins, minerals (electrolytes), flavors including synthetic and natural flavors, colorants, extenders (cheese, chocolate, etc.), pectic acid and salts thereof, alginic acid and salts thereof, organic acids, protective colloidal thickeners, pH control agents, stabilizers, preservatives, glycerin, alcohols, carbonating agents used in carbonated drinks, etc. And, the functional food composition of the present disclosure may comprise pulps used to prepare natural fruit juice, fruit juice drinks and vegetable drinks. These ingredients may be used independently or in combination. The content of these additives is of no great importance. Usually, they are comprised within a range of about 0-20 parts by weight based on 100 parts by weight of the composition of the present disclosure.

Hereinafter, the present disclosure will be described in detail through examples and test examples. However, the following examples and test examples are for illustrative purposes only and the scope of the present disclosure is not limited by the examples and test examples.

[Example 1] Preparation of alpine wormwood (*Artemisia umbelliformis*) extract

The flower of alpine wormwood (*Artemisia umbelliformis*) was harvested, hot air dried and pulverized into powder. The resulting powder was extracted with an ethanol/water solution and then the ethanol was removed through vacuum distillation. After adding glycerin and a preservative to the resulting concentrate, an alpine wormwood (*Artemisia*

*umbelliformis*) extract was obtained by filtration. The alpine wormwood (*Artemisia umbelliformis*) extract (ALPAFLOR® ARTEMISA AO) prepared according to the above-described method was purchased from DSM Nutritional Products Ltd. (4002 Basel, Switzerland) for use in the following test examples.

[Test Example 1] Measurement of melanin

(1) Cell culturing

Melanocytes (KaMC) (KCTC12015BP) deposited in the Korean Collection for Type Culture (KCTC) were cultured in M-254 medium (Gibco BRL, NY, USA) supplemented with Human Melanocyte Growth Supplement (HMGS; Gibco BRL, NY, USA) on a 6-well plate, while changing the medium every other day, in a 5% $CO_2$ incubator at 37 °C until the cells occupied 95% of the plate area (95% confluence).

(2) Treatment with extract

When the density of the melanocytes on the 6-well plate reached about 80% (-80% confluence), they were incubated for a total of 5 days with 10 nM of a pigmentation-inducing substance (endothelin 1, EDN1) and the alpine wormwood (*Artemisia umbelliformis*) extract of Example 1 of different concentrations (0, 0.1, 1 and 2 ppm). The cells not treated with the pigmentation-inducing substance and the alpine wormwood (*Artemisia umbelliformis*) extract of Example 1 were used as a control group.

(3) Recovery of cells and quantification of proteins

400 μL of an Accutase solution (Millipore, CA, USA) was added to the melanocytes (KaMC) cultured on the 6-well plate. After agitation for 1 minute, a total of 1 mL of a cell-containing medium was recovered by adding 600 μL of M-254 medium. Then, after centrifugation at 12,000 rpm for 15 minutes, the supernatant was discarded and 20 μL of MCL lysis buffer (Sigma-Aldrich, St. Louis, USA) was added to the remaining pellets. After mixing well and centrifuging again at 12,000 rpm for 15 minutes, the supernatant and the cell debris were separated.

After adding 1 μL of the separated supernatant to a 96-well plate, distilled water was added to make the final volume 25 μL. Then, the quantity of proteins were determined at 562 nm using the BCA protein assay kit (Thermo Scientific, IL, USA). The result is shown in Table 1.

[Table 1]

| Sample | | Mean (μg/ μL) | Standard deviation |
|---|---|---|---|
| Pigmentation-inducing substance (endothelin 1) (nM) | Alpine wormwood (*Artemisia umbelliformis*) extract (ppm) | | |
| 0 | 0 | 3.949878 | 0.119235 |
| 10 | 0 | 4.452843 | 0.082231 |
| | 0.1 | 4.287126 | 0.20969 |
| | 1 | 4.292941 | 0.037004 |
| | 2 | 4.61856 | 0.160351 |

(4) Melanin assay

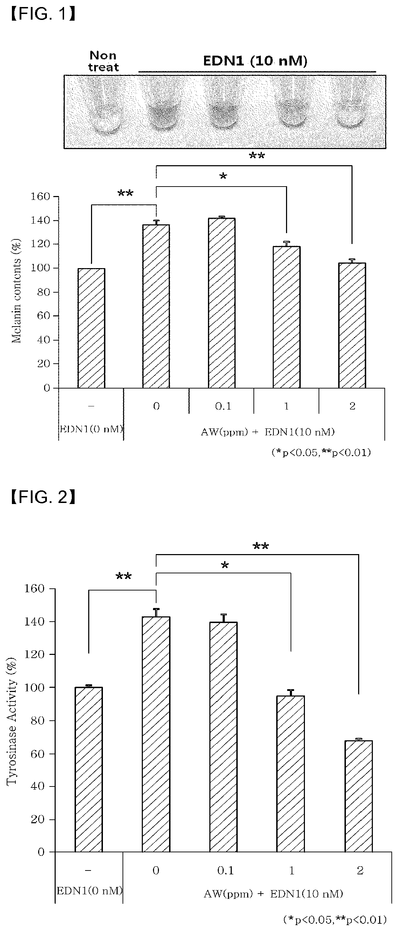

The cell debris separated in (3) of Test Example 1 were lysed at 55 °C for 15 minutes after adding 70 μL of 1 N NaOH (Sigma-Aldrich, St. Louis, USA) and absorbance was measured at 475 nm after transferring to two 96-well plates, 30 μL each. FIG. 1 shows a photograph taken before the measurement and a result of calculating the melanin content from the quantitated protein amount according to Equation 1.

[Equation 1]

Melanin content (%) = (Absorbance / Total protein) x 100

As seen from FIG. 1, when the cells were treated with the alpine wormwood (*Artemisia umbelliformis*) extract of an aspect of the present disclosure, the melanin content which had been increased by the pigmentation-inducing substance (EDN1) was decreased. In particular, when 2 ppm of the alpine wormwood (*Artemisia umbelliformis*) extract was treated, the melanin content decreased to about 104%, comparable to that of the control group. This result could also be perceived visually before the absorbance was measured. That is to say, the color of the test tube was brighter as the concentration of the alpine wormwood (*Artemisia umbelliformis*) extract increased. When the concentration was 2 ppm, the test tube was as clear as the control group. Accordingly, it was confirmed that the alpine wormwood (*Artemisia umbelliformis*) extract of the present disclosure exhibits an effect of reducing the melanin content in cells and thus it can exhibit the effect of whitening skin or treating pigmentation.

[Test Example 2] Inhibition of tyrosinase activity

Based on the result of protein quantification in (3) of Test Example 1, proteins of the amount contained in the supernatant, i.e. 20 $\mu$g, were added to a 96-well plate and PBS (phosphate-buffered saline, without CaCl$_2$ & MgCl$_2$, Welgene, Korea) was added to make the final volume 100 $\mu$L. Then, 100 $\mu$L of 2 mg/mL L-DOPA (L-3,4-dihydroxyphenylalanine) (Sigma-Aldrich, St. Louis, USA) dissolved in PBS was further added. The quantity of dopachrome produced from L-DOPA by the action of tyrosinase was compared by measuring absorbance. After the addition of L-DOPA, absorbance was measured at 475 nm with 10-minute intervals while incubating at 37 °C. FIG. 2 shows a result of calculating the tyrosinase content from the amount of proteins quantitated in (3) of Test Example 1 according to Equation 2.

[Equation 2]

$$\text{Tyrosinase content (\%)} = (\text{Absorbance} / \text{Total protein}) \times 100$$

As seen from FIG. 2, when the cells were treated with the alpine wormwood (*Artemisia umbelliformis*) extract of an aspect of the present disclosure, the tyrosinase activity which had been increased by the pigmentation-inducing substance was decreased. In particular, when 1 ppm of the alpine wormwood (*Artemisia umbelliformis*) extract was treated, the tyrosinase activity decreased to about 95%, comparable to that of the control group. When the concentration of the alpine wormwood (*Artemisia umbelliformis*) extract was 2 ppm, the tyrosinase activity decreased to about 68%. It was lower than that of the control group.

Accordingly, it was confirmed that the alpine wormwood (*Artemisia umbelliformis*) extract of the present disclosure exhibits a remarkable effect of decreasing tyrosinase activity and thus it can exhibit the effect of reducing melanin production, whitening skin or treating pigmentation.

Hereinafter, formulation examples of the composition according to an aspect of the present disclosure will be described. However, other formulations are also possible and the scope of the present disclosure is not limited by the formulation examples.

[Formulation Example 1] Soft capsule

8 mg of the alpine wormwood (*Artemisia umbelliformis*) extract of Example 1, 9 mg of vitamin E, 9 mg of vitamin C, 2 mg of palm oil, 8 mg of hydrogenated vegetable oil, 4 mg of yellow beeswax and 9 mg of lecithin were mixed according to a commonly employed method and a soft capsule was prepared by filling 400 mg of the mixture per capsule. Separately from this, a soft capsule sheet was prepared from 66 parts by weight of gelatin, 24 parts by weight of glycerin and 10 parts by weight of sorbitol, which was then filled with the mixture to prepare a soft capsule in which 400 mg of the composition according to the present disclosure is contained.

[Formulation Example 2] Tablet

8 mg of the alpine wormwood (*Artemisia umbelliformis*) extract of Example 1, 9 mg of vitamin E, 9 mg of vitamin C, 200 mg of galactooligosaccharide, 60 mg of lactose and 140 mg of maltose were mixed and granulated using a fluidized-bed dryer. Then, after adding 6 mg of sugar ester, the mixture was prepared into a tablet containing 500 mg of the composition according to a commonly employed method.

[Formulation Example 3] Drink

8 mg of the alpine wormwood (*Artemisia umbelliformis*) extract of Example 1, 9 mg of vitamin E, 9 mg of vitamin C, 10 g of glucose, 0.6 g of citric acid and 25 g of oligosaccharide syrup were mixed and 300 mL of purified water was added. 200 mL of the mixture was filled in a bottle. Then, a drink was prepared by sterilizing at 130 °C for 4-5 seconds.

[Formulation Example 4] Granule

8 mg of the alpine wormwood (*Artemisia umbelliformis*) extract of Example 1, 9 mg of vitamin E, 9 mg of vitamin C, 250 mg of anhydrous crystalline glucose and 550 mg of starch were mixed and granulated using a fluidized-bed granulator. The resulting granule was filled in a pouch.

[Formulation Example 5] Injection

An injection was prepared according to a commonly employed method with the composition described in Table 2.

[Table 2]

| Ingredient | Content |
|---|---|
| Alpine wormwood (*Artemisia umbelliformis*) extract of Example 1 | 10-50 mg |
| Sterilized distilled water for injection | adequate |
| pH control agent | adequate |

[Formulation Example 6] Health functional food

A health functional food was prepared according to a commonly employed method with the composition described in Table 3.

[Table 3]

| Ingredient | Content |
|---|---|
| Alpine wormwood (*Artemisia umbelliformis*) extract of Example 1 | 20 mg |
| Vitamin A acetate | 70 $\mu$g |
| Vitamin E | 1.0 mg |
| Vitamin $B_1$ | 0.13 mg |
| Vitamin $B_2$ | 0.15 mg |
| Vitamin $B_6$ | 0.5 mg |
| Vitamin $B_{12}$ | 0.2 $\mu$g |
| Vitamin C | 10 mg |
| Biotin | 10 $\mu$g |
| Nicotinamide | 1.7 mg |
| Folic acid | 50 $\mu$g |
| Calcium pantothenate | 0.5 mg |
| Ferrous sulfate | 1.75 mg |
| Zinc oxide | 0.82 mg |
| Magnesium carbonate | 25.3 mg |
| Potassium dihydrogen phosphate | 15 mg |
| Calcium monohydrogen phosphate | 55 mg |
| Potassium citrate | 90 mg |
| Calcium carbonate | 100 mg |
| Magnesium chloride | 24.8 mg |

Although specific compositions of vitamin and mineral mixtures suitable for a health functional food were described as above, the compositions may be changed as desired.

[Formulation Example 7] Health drink

A health drink was prepared according to a commonly employed method with the composition described in Table 4.

[Table 4]

| Ingredient | Content |
|---|---|
| Alpine wormwood (*Artemisia umbelliformis*) extract of Example 1 | 1000 mg |
| Citric acid | 1000 mg |
| Oligosaccharide | 100 g |
| Taurine | 1 g |
| Purified water | balance |

According to a commonly employed health drink preparation method, the above-described ingredients were mixed and heated at 85 °C for about 1 hour under agitation. The resulting solution was filtered and sterilized.

[Formulation Example 8] Softening lotion (skin lotion)

A softening lotion was prepared according to a commonly employed method with the composition described in Table 5.

[Table 5]

| Ingredient | Content (wt%) |
|---|---|
| Alpine wormwood (*Artemisia umbelliformis*) extract of Example 1 | 0.2 |
| Glycerin | 3.0 |
| Butylene glycol | 2.0 |
| Propylene glycol | 2.0 |
| Carboxyvinyl polymer | 0.1 |
| PEG-12 nonyl phenyl ether | 0.2 |
| Polysorbate 80 | 0.4 |
| Ethanol | 10.0 |
| Triethanolamine | 0.1 |
| Preservative, pigment and flavor | adequate |
| Purified water | balance |

[Formulation Example 9] Nourishing lotion (milk lotion)

A nourishing lotion was prepared according to a commonly employed method with the composition described in Table 6.

[Table 6]

| Ingredient | Content (wt%) |
|---|---|
| Alpine wormwood (*Artemisia umbelliformis*) extract of Example 1 | 1.0 |
| Glycerin | 3.0 |

(continued)

| Ingredient | Content (wt%) |
|---|---|
| Butylene glycol | 3.0 |
| Propylene glycol | 3.0 |
| Carboxyvinyl polymer | 0.1 |
| Beeswax | 4.0 |
| Polysorbate 60 | 1.5 |
| Caprylic/capric triglyceride | 5.0 |
| Squalane | 5.0 |
| Sorbitan sesquioleate | 1.5 |
| Liquid paraffin | 0.5 |
| Cetearyl alcohol | 1.0 |
| Triethanolamine | 0.2 |
| Preservative, pigment and flavor | adequate |
| Purified water | balance |

[Formulation Example 10] Nourishing cream

A nourishing cream was prepared according to a commonly employed method with the composition described in Table 7.

[Table 7]

| Ingredient | Content (wt%) |
|---|---|
| Alpine wormwood (*Artemisia umbelliformis*) extract of Example 1 | 2.0 |
| Glycerin | 3.0 |
| Butylene glycol | 3.0 |
| Liquid paraffin | 7.0 |
| β-Glucan | 7.0 |
| Carbomer | 0.1 |
| Caprylic/capric triglyceride | 3.0 |
| Squalane | 5.0 |
| Cetearyl glucoside | 1.5 |
| Sorbitan stearate | 0.4 |
| Polysorbate 60 | 1.2 |
| Triethanolamine | 0.1 |
| Preservative, pigment and flavor | adequate |
| Purified water | balance |

[Formulation Example 11] Massage cream

A massage cream was prepared according to a commonly employed method with the composition described in Table 8.

[Table 8]

| Ingredient | Content (wt%) |
|---|---|
| Alpine wormwood (*Artemisia umbelliformis*) extract of Example 1 | 2.0 |
| Glycerin | 8.0 |
| Butylene glycol | 4.0 |
| Liquid paraffin | 45.0 |
| β-Glucan | 7.0 |
| Carbomer | 0.1 |
| Caprylic/capric triglyceride | 3.0 |
| Beeswax | 4.0 |
| Cetearyl glucoside | 1.5 |
| Sorbitan sesquioleate | 0.9 |
| Vaseline | 3.0 |
| paraffin | 1.5 |
| Preservative, pigment and flavor | adequate |
| Purified water | balance |

[Formulation Example 12] Pack

A pack was prepared according to a commonly employed method with the composition described in Table 9.

[Table 9]

| Ingredient | Content (wt%) |
|---|---|
| Alpine wormwood (*Artemisia umbelliformis*) extract of Example 1 | 0.2 |
| Glycerin | 4.0 |
| Polyvinyl alcohol | 15.0 |
| Hyaluronic acid extract | 5.0 |
| β-Glucan | 7.0 |
| Allantoin | 0.1 |
| Nonyl phenyl ether | 0.4 |
| Polysorbate 60 | 1.2 |
| Ethanol | 6.0 |
| Preservative, pigment and flavor | adequate |
| Purified water | balance |

Accordingly, it is to be understood that the substantial scope of the present disclosure is defined by the appended claims.

[Accession Number]

Depository institution: Korea Research Institute of Bioscience and Biotechnology.

Accession number: KCTC12015 BP.
Date of accession: Sep. 14, 2011.

## Claims

1. Non-therapeutic cosmetic use of an alpine wormwood (*Artemisia umbelliformis*) extract as an active ingredient for skin whitening, inhibiting melanin production, reducing melanin, or suppressing pigmentation.

2. The use according to claim 1, wherein the alpine wormwood (*Artemisia umbelliformis*) is one or more selected from a group consisting of the leaf, flower, stem and root of the plant alpine wormwood (*Artemisia umbelliformis*).

3. The use according to any of claims 1 to 2, wherein the extract is comprised in a composition, wherein the concentration of the alpine wormwood (*Artemisia umbelliformis*) extract is 1 ppm or higher based on the total volume of the composition.

4. The use according to any of claims 1 to 3, wherein the alpine wormwood (*Artemisia umbelliformis*) extract is an extract of one or more selected from a group consisting of a water, an organic solvent and a mixture thereof.

5. The use according to claim 4, wherein the organic solvent is one or more selected from a group consisting of a $C_1$-$C_6$ lower alcohol, butylene glycol and propylene glycol.

6. The use according to claim 5, wherein the lower alcohol is ethanol.

7. The use according to any of claims 1 to 6, wherein the extract is comprised in a composition, wherein the composition is a pharmaceutical, food or cosmetic composition.

## Patentansprüche

1. Nicht therapeutische kosmetische Verwendung eines Extrakts von Silberraute (*Artemisia umbelliformis*) als einen Wirkstoff für die Hautaufhellung, der die Erzeugung von Melanin verhindert, Melanin reduziert oder die Pigmentierung unterdrückt.

2. Verwendung nach Anspruch 1, wobei die Silberraute (*Artemisia umbelliformis*) eines oder mehrere ist, ausgewählt aus der Gruppe, bestehend aus dem Blatt, der Blume, dem Stamm und der Wurzel der Silberrautenpflanze (*Artemisia umbelliformis*).

3. Verwendung nach einem der Ansprüche 1 bis 2, wobei der Extrakt in einer Zusammensetzung enthalten ist, wobei die Konzentration des Extrakts von Silberraute (*Artemisia umbelliformis*) 1 ppm oder höher ist, basierend auf dem Gesamtvolumen der Zusammensetzung.

4. Verwendung nach einem der Ansprüche 1 bis 3, wobei der Extrakt von Silberraute (*Artemisia umbelliformis*) ein Extrakt von einem oder mehreren ist, ausgewählt aus der Gruppe, bestehend aus einem Wasser, einem organischen Lösemittel und einem Gemisch daraus.

5. Verwendung nach Anspruch 4, wobei das organische Lösemittel eines oder mehrere ist, ausgewählt aus der Gruppe, bestehend aus einem $C_1$-$C_6$-Niederalkkohol, Butylenglykol und Propylenglykol.

6. Verwendung nach Anspruch 5, wobei der Niederalkohol Ethanol ist.

7. Verwendung nach einem der Ansprüche 1 bis 6, wobei der Extrakt in einer Zusammensetzung enthalten ist, wobei die Zusammensetzung eine pharmazeutische, Lebensmittel- oder kosmetische Zusammensetzung ist.

## Revendications

1. Utilisation cosmétique non thérapeutique d'un extrait de Genépi jaune (*Artemisia umbelliformis*) comme ingrédient actif pour blanchir la peau, inhiber la production de mélanine, réduire la mélanine ou supprimer la pigmentation.

2. Utilisation selon la revendication 1, dans laquelle le Genépi jaune (*Artemisia umbelliformis*) est une ou plusieurs choisies dans le groupe constitué par la feuille, la fleur, la tige et la racine de la plante Genépi jaune (*Artemisia*

*umbelliformis*).

**3.** Utilisation selon l'une quelconque des revendications 1 à 2, dans laquelle l'extrait est compris dans une composition, dans laquelle la concentration de l'extrait de Genépi jaune (*Artemisia umbelliformis*) est de 1 ppm ou plus par rapport au volume total de la composition.

**4.** Utilisation selon l'une quelconque des revendications 1 à 3, dans laquelle l'extrait de Genépi jaune (*Artemisia umbelliformis*) est un extrait d'un ou plusieurs choisis dans un groupe constitué par de l'eau, un solvant organique et un mélange de ceux-ci.

**5.** Utilisation selon la revendication 4, dans laquelle le solvant organique est un ou plusieurs solvants choisis dans un groupe constitué par un alcool inférieur en C1-C6, le butylèneglycol et le propylèneglycol.

**6.** Utilisation selon la revendication 5, dans laquelle l'alcool inférieur est l'éthanol.

**7.** Utilisation selon l'une quelconque des revendications 1 à 6, dans laquelle l'extrait est compris dans une composition, dans laquelle la composition est une composition pharmaceutique, alimentaire ou cosmétique.

【FIG. 1】

(*p<0.05,**p<0.01)

【FIG. 2】

(*p<0.05,**p<0.01)

**REFERENCES CITED IN THE DESCRIPTION**

**Patent documents cited in the description**

- CH 696135
- WO 2009088109 A
- KR 1020140037504
- KR 2012007815 W
- KR 1020110099728